# EUROPÄISCHE PATENTSCHRIFT

(11) **EP 1 898 964 B1**
(45) Veröffentlichungstag und Bekanntmachung des Hinweises auf die Patenterteilung: **05.09.2012**
(21) Anmeldenummer: 06762224.1
(22) Anmeldetag: 28.06.2006
(51) Int. Cl.: A61L 2/20, A61L 2/24, B01D 1/00, B01D 1/16, F22B 1/28

(54) **VERFAHREN ZUM ÜBERWACHEN EINES VERDAMPFERS**
METHOD FOR MONITORING AN EVAPORATOR
PROCEDE POUR SURVEILLER UN DISPOSITIF D'EVAPORATION

(30) Priorität: 01.07.2005 DE 102005030822
(43) Veröffentlichungstag der Anmeldung: 19.03.2008
(73) Patentinhaber: Krones AG, 93073 Neutraubling (DE)
(72) Erfinder: MEINZINGER, Rupert, 94356 Kirchroth (DE); ZIEGLER, Manfred, 94161 Ruderting (DE)
(86) Internationale Anmeldenummer: PCT/EP2006/006226
(87) Internationale Veröffentlichungsnummer: WO 2007/003313

(56) Entgegenhaltungen:
- WO-A-90/07366
- WO-A-98/31844
- WO-A-03/082355
- DE-A1- 19 704 639
- US-A- 5 290 511
- US-A1- 2004 182 855
- PATENT ABSTRACTS OF JAPAN Bd. 017, Nr. 225 (C-1055), 10. Mai 1993 (1993-05-10) -& JP 04 361754 A (TABAI ESPEC CORP), 15. Dezember 1992 (1992-12-15)

## Beschreibung

Die Erfindung betrifft ein Verfahren zum Überwachen eines Verdampfers, insbesondere für eine Desinfektionsflüssigkeit zum Sterilisieren von Behältern, gemäß dem Oberbegriff des Anspruchs 1.

Es ist bereit bekannt, Desinfektionsflüssigkeiten, z. B. Wasserstoffperoxid, durch dosiertes Auftropfen auf einen erwärmten Heizkörper mehr oder weniger vollständig zu verdampfen und den so erzeugten Dampf, ggf. nach Vermischung mit Heißluft, den zu sterilisierenden Behältern wie z. B. Bechern oder Flaschen zuzuführen (DE 37 28 595 A1, US 5,879,648).

Ferner ist es bekannt, die Temperatur eines Heizkörpers zum Verdampfen von Flüssigkeiten mittels Sensoren abzutasten und so die Heizelemente für den Heizkörper, z. B. elektrische Heizpatronen, zu regeln (WO 90/07366, DE 197 04 639 A1). Hierdurch wird zwar die Einhaltung der gewünschten Heizkörpertemperatur verbessert; es lässt sich jedoch nicht feststellen, ob die vorgegebene Menge an Flüssigkeit auch tatsächlich zugeführt bzw. verdampft worden ist. Dies ist besonders nachteilig, wenn mit dem erzeugten Dampf Behälter für Lebensmittel sterilisiert werden, da hierbei mehr und mehr eine exakte Kontrolle der Sterilisationswirkung bis hin zur Protokollierung aller maßgeblichen Werte für jeden einzelnen Behälter gefordert wird.

Es wurde auch schon vorgeschlagen, in einer von einem Verdampfer beaufschlagten Sterilisationskammer für Behälter mittels Sensoren z. B. Temperatur, Feuchtigkeit, Dampfkonzentration, Strömung oder Druck zu messen (EP 1 368 068 B1). Diese Überwachung ist äußerst aufwendig und von begrenzter Aussagekraft hinsichtlich der Menge des verdampften Sterilisationsmittels.

Durch die WO 03/082355 ist eine Sterilisationsvorrichtung für Luft bekannt, die über ein Ende die zu sterilisierende Luft ansaugt, anwärmt und mit über einen Verdampfer geleite Sterilisationsflüssigkeit zur Sterilisation derselben versieht und über ein zweites Ende die sterilisierte Luft über eine Verteilerdüse abgibt und verteilt.

Aus der WO 98/31844 ist ebenfalls ein Verdampfer für ein zu verdampfendes Medium bekannt. Dabei weist der Verdampfer eine Verdampferkammer einen Dom und eine Auftropfdüse auf, mit der das zu verdampfende Medium auf den Dom gebracht werden kann. Der Dom weist zusätzlich eine Heizung auf. Um die Funktion des Verdampfers zu überwachen, lehrt die WO98/31844 die Anbringung von Temperatursensoren innerhalb und außerhalb des Doms.

Der Erfindung liegt die Aufgabe zugrunde, ein Verfahren zum Überwachen eines Verdampfers der eingangs genannten Art zu schaffen, das einfach durchzuführen ist und eine direkte Kontrolle der verdampften Flüssigkeit erlaubt. Außerdem soll eine kostengünstige und funktionssichere Vorrichtung zur Durchführung des Verfahrens geschaffen werden.

Diese Aufgabe wird hinsichtlich des Verfahrens durch die Merkmale des Anspruchs 1 und hinsichtlich der Vorrichtung durch die Merkmale des Anspruchs 6 gelöst.

Durch die erfindungsgemäße Kontrolle der durch die auftreffende Flüssigkeit vom Heizkörper abgezogenen Verdampfungswärme können auf einfache Weise sowohl Störungen in der Flüssigkeitszufuhr, z. B. durch verstopfte Düsen oder Ventile, als auch Störungen beim Verdampfungsvorgang, z. B durch ungenaues Auftropfen oder verschmutzte Oberflächen zuverlässig erkannt werden. Es hat sich nämlich überraschender Weise gezeigt, dass beim Auftröpfeln bereits weniger ml Flüssigkeit, z. B. Wasserstoffperoxid in wässriger Lösung, auf einen Heizkörper mit einer Temperatur von z. B. 180° C ein abrupter Temperaturabfall auf beispielsweise 160° C auftritt, der mittels herkömmlicher Temperaturfühler problemlos erfasst werden kann.

Eine besonders exakte Erfassung des Temperaturabfalls ist möglich, wenn gemäß einer Weiterbildung der Erfindung die Temperatur des Heizkörpers im Auftropfbereich der Flüssigkeit gemessen wird.

Eine weitere Steigerung der Genauigkeit ist möglich, wenn gemäß einer anderen Weiterbildung der Erfindung zusätzlich die Temperatur des Heizkörpers außerhalb des Auftropfbereichs der Flüssigkeit gemessen und mit der Temperatur im Auftropfbereich in Relation gesetzt wird. Auf diese Weise kann der Temperaturabfall in Form einer Temperaturdifferenz gemessen werden, wobei die Grundtemperatur des Heizkörpers und deren Schwankungen kompensiert werden können.

Auch kann gemäß einer anderen vorteilhaften Weiterbildung der Erfindung die verdampfte Flüssigkeitsmenge ermittelt werden, indem der zeitliche Verlauf des Temperaturabfalls erfasst wird.

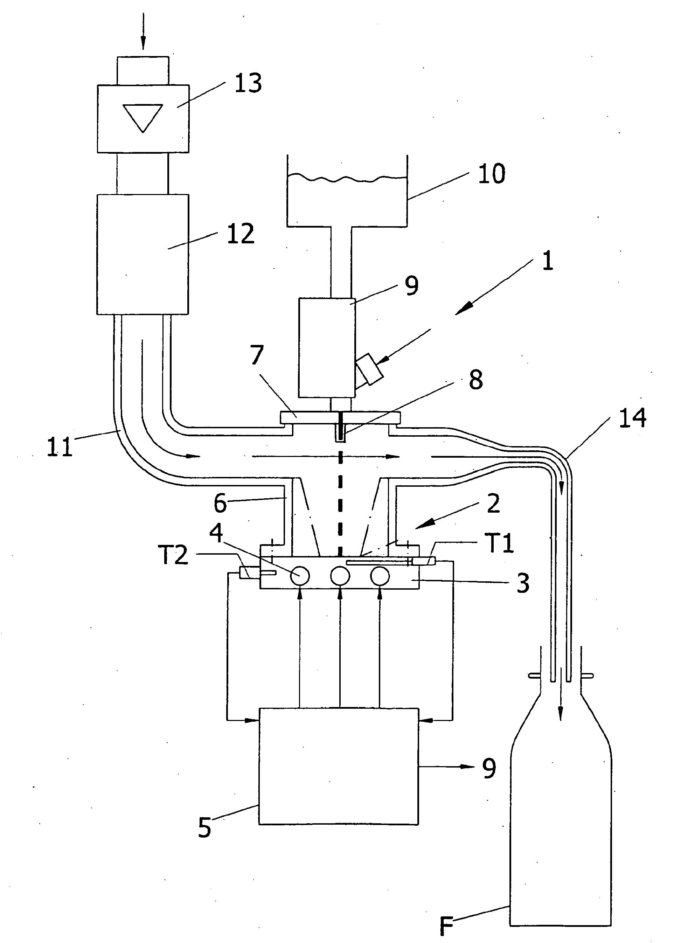

Im Nachstehenden wird ein Ausführungsbeispiel der Erfindung anhand der Zeichnung beschrieben. Diese zeigt die schematische Seitenansicht einer Vorrichtung 1 zum Sterilisieren von Gefäßen F in Form von PET-Flaschen für Getränke mit einem Verdampfer 2 für Wasserstoffperoxid in wässriger Lösung.

Der Verdampfer 2 weist einen metallischen Heizkörper 3 mit integrierten elektrischen Heizpatronen 4 auf, deren Energieversorgung durch ein elektronisches Steuergerät 5 geregelt wird. An dieses sind zwei Temperaturfühler T1 und T2 angeschlossen, wobei der Messbereich des ersten Temperaturfühlers T1 mittig in dem Heizkörper 3 und damit innerhalb der eigentlichen Verdampfungszone und der Messbereich des zweiten Temperaturfühlers T2 außermittig im Heizkörper 3 und damit außerhalb der Verdampfungszone angeordnet ist. Mit Hilfe des zweiten Temperaturfühlers T2 hält das Steuergerät 5 den Heizkörper 3 auf der gewünschten Verdampfungstemperatur von beispielsweise 180°C.

Der Verdampfer 2 umfasst ferner ein topfartiges Gehäuse 6, dessen Boden durch den lösbar befestigten, plattenförmigen Heizkörper 3 gebildet wird. Der Deckel 7 des Gehäuses 6 ist gleichfalls zu Reinigungszwecken lösbar befestigt und trägt eine Einspritzdüse 8, deren Öffnung senkrecht nach unten auf die Mitte des Heizkörpers 3 gerichtet ist und so die eigentliche Verdämpfungszone definiert. Die Einspritzdüse 8 wird über ein elektromagnetisch betätigtes Ventil 9 und einen Vorratstank 10 mit einer wässrigen Lösung mit ca. 30 % Wasserstoffperoxid versorgt. Das Ventil 9 wird durch das Steuergerät 5 betätigt.

An das Gehäuse 6 ist seitlich eine erste Rohrleitung 11 mit einem Erhitzer 12 und einem Gebläse 13, erforderlichenfalls mit einem nicht gezeigten Sterilfilter und einem nicht gezeigten Trockner, angeschlossen, durch das auf eine Temperatur von mehr als 100° C erwärmte, trockene und sterile Luft von der Seite her mit Abstand zum Heizkörper 3 in das Gehäuse 6 eingeleitet wird. An der der ersten Rohrleitung 11 gegenüberliegenden Seite ist eine zweite Rohrleitung 14 angeordnet, durch welche die durch die Rohrleitung 11 einströmende, mit dem auf der Heizplatte 3 verdampften Wasserstoffperoxid vermischte Luft das Gehäuse 6 wieder verlässt und in die zu sterilisierende Flasche F eingeleitet wird.

Der erste Temperaturfühler T1 ist, wie bereits gesagt, mittig im Heizkörper 3 und zwar mit geringem Abstand zu dessen horizontaler Oberfläche, die die eigentliche Verdampfungsfläche bildet, angeordnet. Er ist daher in der Lage, die Temperatur der Verdampfungsfläche exakt zu erfassen und diese an das Steuergerät 5 zu melden. Dieses ist durch den zweiten Temperaturfühler T2 über die Grundtemperatur des Heizkörpers 3 von z. B. 180° C informiert, die es weitgehend konstant hält.

Ist ein zu sterilisierendes Gefäß F an die zweite Rohrleitung 14 angeschlossen, so löst das Steuergerät 5 (z. B. in Verbindung mit der Zentralsteuerung einer rotierenden Sterilisationsmaschine mit mehreren Verdampfern 2 und mehreren Leitungen 14) durch kurzzeitiges (z. B. 5 sec.) Öffnen des Ventils 9 das Auftröpfeln einer vorgegebenen Menge von z. B. 1 ml Wasserstoffperoxidlösung auf den Heizkörper 3 aus. An dessen Verdampfungsfläche wird die Wasserstoffperoxidlösung schlagartig in die Dampfphase übergeführt, mit der durch die erste Rohrleitung 11 eingeblasenen Heißluft vermischt und in die zweite Rohrleitung 14 mitgerissen, von der es in die Flasche F eingeleitet wird. Gleichzeitig mit dem Verdampfungsvorgang fällt die Temperatur im Verdampfungsbereich um beispielsweise 20° C ab, was durch den ersten Temperaturfühler T1 erfasst und an das Steuergerät 5 gemeldet wird. Dort wird das Steuersignal zum Öffnen des Ventils 9 mit dem Temperatursignal des Temperaturfühlers T1 korreliert. Findet der vorbeschriebene momentane Temperaturabfall während und/oder kurz nach dem Auftröpfeln der Wasserstoffperoxidlösung auf den Heizkörper 3 statt, so wird der Verdampfungsvorgang und damit der Sterilisationsvorgang für das jeweilige Gefäß F als in Ordnung befunden und evtl. entsprechend protokolliert. Findet der vorbeschriebene Temperaturabfall nicht statt oder überschreitet er nicht einen vorgegebenen Schwellwert von z. B. 20° C, so wird der Verdampfungsvorgang und somit der Sterilisationsvorgang als nicht ausreichend bzw. fehlerhaft beurteilt und beispielsweise ein Signal erzeugt, das zum Aussortieren des betreffenden Gefäßes F sowie zu einem Warnsignal führt.

Der Schwellwert wird vorzugsweise mit dem vom Temperatursensor T2 gemessenen Basiswert für die Heizkörpertemperatur verknüpft und auf beispielsweise 20° C Temperaturdifferenz eingestellt, so dass Schwankungen in der Grundtemperatur des Heizkörpers 3 automatisch kompensiert werden.

Der vorbeschriebene Temperaturabfall ist relativ kurzzeitig, da die Verdampfungswärme sofort durch die im Verdampfer 2 nachfließende, von den Heizpatronen 4 gelieferte Wärme ersetzt wird. Da diese Parameter im Wesentlichen konstant sind, kann aus dem zeitlichen Verlauf des Temperaturabfalls auch die Menge der entnommenen Verdampfungswärme und daraus wiederum die Menge des verdampften Wasserstoffperoxids ermittelt und ggf. protokolliert werden. Dies ist insofern von Vorteil, als die bei einem Sterilisationsvorgang für eine Flasche F erforderliche Menge von Wasserstoffperoxidlösung sehr klein ist und daher auf andere Weise, z. B. mittels Durchflussmessung, nur sehr schwer zu erfassen ist. Dem gegenüber ermöglicht das erfindungsgemäße Verfahren auf einfache Weise eine vollständige Kontrolle des Verdampfungsvorgangs.

Erfolgt nach der z. B. einige Sekunden dauernden Verdampfungsphase eine gleichfalls z. B. einige Sekunden dauernde "Ruhephase" des Heizkörpers 3 bei geschlossenem Ventil 9, während der beispielsweise der Wasserstoffperoxiddampf aus der Flasche F ausgespült wird, so kann ein einziger Temperaturfühler T1 auch die Regelung der Heizpatrone 4 übernehmen. Er überwacht dann den Temperaturanstieg des Heizkörpers 3 während dieser Ruhephase.

Der Heizkörper 3 kann auch topfartig ausgebildet sein, wie in der Zeichnung strichpunktiert angedeutet ist.

## Patentansprüche

1. Verfahren zum Überwachen eines Verdampfers, bei dem auf einen erwärmten Heizkörper mittels Einspritzdüse eine Flüssigkeit aufgebracht und verdampft wird, wobei der durch die Verdampfungswärme im Heizkörper hervorgerufene Temperaturabfall erfasst wird, **dadurch gekennzeichnet, dass** die Temperatur des Heizkörpers im Auftropfbereich der Flüssigkeit gemessen wird und dass zusätzlich die Temperatur des Heizkörpers außerhalb des Auftropfbereichs der Flüssigkeit gemessen und mit der Temperatur im Auftropfbereich in Relation gesetzt wird und dass der zeitliche Verlauf des Temperaturabfalls erfasst und daraus die verdampfte Flüssigkeitsmenge ermittelt wird.

2. Verfahren nach Anspruch 1, **dadurch gekennzeichnet, dass** bei Überschreiten eines vorgegebenen Temperaturabfalls ein Signal erzeugt wird.

## Claims

1. Method for monitoring an evaporator, in which a liquid is applied to a heated heater by means of a spray nozzle and evaporated, in which the fall in temperature produced in the heater by heat evaporation is detected, **characterised in that** the temperature of the heater in the dropping area of the liquid is measured and the temperature of the heater is also measured outside the dropping area of the liquid and related to the temperature in the dropping area and the time taken for the fall in temperature is detected and from this the amount of liquid evaporated is determined.

2. Method according to claim 1, **characterised in that** a signal is produced when a preset fall in temperature is exceeded.

## Revendications

1. Procédé pour surveiller un évaporateur selon lequel on transfère et vaporise un liquide sur un élément thermique chauffé au moyen de buses d'injection, et on détecte la chute de température provoquée dans l'élément thermique par la chaleur de vaporisation,
**caractérisé en ce que**
l'on mesure la température de l'élément thermique dans la zone d'application des gouttes de liquide, on mesure en outre la température de l'élément thermique à l'extérieur de la zone d'application des gouttes de liquide et on la met en rapport avec la température dans la zone d'application des gouttes, on détermine les variations chronologiques de la chute de température et on en déduit la quantité de liquide vaporisé.

2. Procédé conforme à la revendication 1,
**caractérisé en ce qu'**
en cas de dépassement d'une chute de température prédéfinie un signal est produit.
